# FASCICULE DE BREVET EUROPEEN

(11) **EP 2 322 135 B1**
(45) Date de publication et mention de la délivrance du brevet: **11.05.2016**
(21) Numéro de dépôt: 10190271.6
(22) Date de dépôt: 05.11.2010
(51) Int. Cl.: A61K 8/81, A61K 8/02, A61K 8/04, A61K 8/73, A61K 8/97, A61Q 5/06, A61Q 5/02, A61Q 19/10, A61Q 5/12

(54) **Composition cosmétique comprenant un agent structurant, un agent absorbant et un tensioactif pour application sur les matières kératiniques**
Kosmetische Zusammensetzung; die eine strukturgebende Substanz, eine absorbierende Substanz und ein Tensid zur Anwendung auf keratinhaltige Stoffe enthält
Cosmetic composition including a structuring agent, an absorbent agent and a surfactant for application to keratin materials

(30) Priorité: 13.11.2009 FR 0958027
(43) Date de publication de la demande: 18.05.2011
(73) Titulaire: L'Oréal, 75008 Paris (FR)
(72) Inventeur: Sellier, Céline, 94140, Alfortville (FR); Gabin, Gérard, 75009, Paris (FR)
(74) Mandataire: Dossmann, Gérard

(56) Documents cités:
- EP-A1- 0 745 378
- EP-A1- 0 745 379
- EP-A1- 0 968 703
- EP-A1- 1 106 165
- FR-A1- 2 555 441
- FR-A1- 2 779 649
- US-A1- 2007 009 456

## Description

La présente invention se rapporte à une composition cosmétique anhydre comprenant un agent structurant organique, un agent absorbant particulier, un agent tensioactif et une phase liquide anhydre, ainsi qu'à l'utilisation de cette composition pour le nettoyage et/ou le conditionnement des matières kératiniques (peau et/ou cheveux).

De nombreux produits de soin capillaire ont été décrits dans l'art antérieur. Les compositions correspondantes ont pour but d'apporter de bonnes propriétés cosmétiques aux cheveux.

Les produits classiques de soins des matières kératiniques et en particulier des cheveux, par exemple les après-shampooings ou les masques, se présentent le plus souvent sous forme de crèmes plus ou moins visqueuses. De même, les produits de nettoyage des matières kératiniques sont le plus souvent sous forme de liquides plus ou moins visqueux.

Mais ces produits ont tendance à s'échapper entre les doigts ou à s'échapper de leur conditionnement, ce qui peut être très gênant lorsqu'ils viennent au contact des vêtements, par exemple lors de déplacements. De plus, ils nécessitent la présence d'un conservateur afin d'assurer la qualité microbiologique du produit.

On connaît du document EP 0 968 703 une composition cosmétique ou dermatologique solide, pouvant être déformable, comprenant un agent structurant, un agent absorbant organique, éventuellement une phase liquide tensioactive. Cette composition peut être utilisée notamment pour le traitement cosmétique des cheveux.

La présente invention a donc pour but de remédier aux inconvénients de l'art antérieur.

Ainsi, la présente invention a pour objet une composition cosmétique anhydre comprenant un ou plusieurs agents structurants organiques, plusieurs agents absorbants choisis parmi les mélanges d'un ou plusieurs amidons modifiés et d'une ou plusieurs farines de bois, un ou plusieurs agents tensioactifs et une phase liquide anhydre.

Par composition anhydre, on entend une composition qui ne contient pas d'eau (0%), ou présente une teneur en eau inférieure à 5% en poids, de préférence inférieure à 2% en poids, et de manière encore plus particulière inférieure à 1% en poids, par rapport au poids total de la composition. Il est à noter que l'eau peut aussi se trouver sous forme d'eau liée, comme l'eau de cristallisation des sels ou des traces d'eau absorbée par les matières premières utilisées dans la réalisation des compositions selon l'invention.

Par phase liquide anhydre, on entend une phase liquide qui ne contient pas d'eau (0%), ou présente une teneur en eau inférieure à 5% en poids, de préférence inférieure à 2% en poids, et de manière encore plus particulière inférieure à 1% en poids, par rapport au poids total de la phase liquide.

La composition selon l'invention présente une texture tout à fait inhabituelle. La texture est de très faible densité, généralement entre 0,3 et 0,8, et de grande douceur au toucher. Elle peut aller de la crème foisonnée au solide modelable non collant plus ou moins ferme.

Le comportement solide de la composition selon l'invention permet une manipulation facile du produit et une facilité de transport. La texture étant très modelable, la composition est très aisément dosable et applicable. La composition peut être facilement fractionnée à la main afin de ne prélever que la quantité nécessaire de produit. En particulier, cette composition peut être conditionnée sous forme de monodose et par exemple sous forme de petits cubes ou de berlingots.

De plus, la composition se délite rapidement et aisément au contact d'un liquide de préférence aqueux.

Par délitage, on entend au sens de la présente invention un délitage à l'aide d'un liquide et non pas un délitage au toucher comme cela est le cas pour certaines compositions de maquillage de type fards à paupières qui peuvent être prélevées au doigt ou à l'aide d'un pinceau. Ce délitage à l'aide d'un liquide correspond en fait à une déstructuration du solide, avec une solubilisation ou une mise en dispersion des particules dans le liquide.

La composition selon l'invention se rince très facilement et donc nécessite assez peu d'eau pour cela.

La composition selon l'invention présente également un grand confort à l'application. En particulier, on ne constate aucune coulure de la composition, contrairement aux compositions classiques, risquant d'irriter notamment le visage et les yeux. L'absence de coulures est très appréciée dans le cas des permanentes et colorations, ainsi que pour les shampooings destinés aux enfants.

La structure de la composition selon l'invention, qui est une matrice encombrée de type granulaire, permet de formuler des produits contenant des matières premières, tels que des corps gras, à des pourcentages importants et notamment supérieurs à 15% en poids de la composition, ce qui n'est pas aisé avec les structures classiques.

De plus, le caractère anhydre de la composition permet d'éviter l'utilisation de conservateurs.

La composition selon l'invention peut également présenter un effet chauffant lors de sa dispersion en milieu aqueux. Elle peut également présenter un effet moussant selon la nature et la concentration de tensioactif(s) introduit(s).

Enfin, la composition selon l'invention permet d'obtenir un toucher doux sur les matières kératiniques et en particulier les cheveux et un effet coiffant sur cheveux séchés.

Le ou les agents structurants (encore appelés agents texturants) organiques présents dans la composition selon l'invention sont de préférence formés d'un ou plusieurs types de particules. De préférence, les particules sont des particules rigides et insolubles dans le milieu. Les agents structurants sont des composés qui ont la caractéristique de pouvoir s'éliminer facilement des supports kératiniques par simple mise en contact avec de l'eau pure ou de l'eau additionnée de composés polaires.

Comme critère de choix de l'agent structurant, on peut réaliser le test suivant :
- mélange des particules testées avec de l'eau contenant un colorant usuel, jusqu'à l'obtention d'une pâte colorée,
- versement d'une goutte d'eau pure ou d'eau additionnée à 10% d'un composé choisi parmi l'éthanol, le propylène glycol ou le laurylsulfate de sodium sur la pâte ainsi préparée.

Lorsque la pâte au point d'impact de la goutte est beaucoup plus claire que le reste de la pâte, cela signifie que les particules considérées sont candidates comme agent structurant; inversement, lorsque la pâte au point d'impact ne s'est pas décolorée, les particules considérées ne sont pas appropriées.

En vue d'obtenir un solide au toucher agréable et doux, il est préférable d'utiliser des particules ayant une granulométrie moyenne de 1 à 300 microns (µm), par exemple de 5 à 200 µm, et de préférence de 10 à 100 µm, et encore mieux de 15 à 40 µm.

Afin de conférer à la composition de l'invention un aspect aéré et léger, on utilise de préférence des particules ayant une densité inférieure à 0,1, notamment inférieure à 0,09 et, mieux, inférieure à 0,06 et, encore mieux inférieure à 0,04.

En vue d'obtenir cette faible densité, on utilise avantageusement des particules creuses éventuellement remplies d'un gaz. Ce gaz peut être notamment de l'air, de l'azote, de l'isobutane, de l'isopentane.

Selon une autre caractéristique avantageuse de l'invention, les particules se présentent sous forme de billes. Il est toutefois possible d'utiliser des particules ayant la forme de fibres ou d'aiguilles.

Ces particules peuvent être réalisées en différents matériaux inertes ne réagissant pas chimiquement avec le milieu; en particulier ces particules ne réagissent pas avec les huiles, les tensioactifs, l'eau et les différents autres constituants de la composition tels que les actifs.

Les particules sont avantageusement choisies parmi les particules de matériaux thermoplastiques choisis parmi les polyamides, tel que le Nylon, les polymères ou copolymères d'acrylonitrile, de chlorure de vinylidène, de chlorure de vinyle et/ou de monomère acrylique ou styrénique, éventuellement expansés, et les microsphères microporeuses.

Le monomère acrylique est par exemple un acrylate ou méthacrylate de méthyle ou d'éthyle. Le monomère styrénique est par exemple l'α-méthyl-styrène ou le styrène.

Comme particules de Nylon, on peut utiliser les particules d'Orgasol vendues par la société Atochem. Ces particules sont des sphères pleines, poreuses, de diamètre allant de 5 µm à 60 µm.

De préférence, les particules sont des particules creuses déformables d'un copolymère expansé de chlorure de vinylidène et d'acrylonitrile, ou de chlorure de vinylidène, d'acrylonitrile et de (méth)acrylate ou monomère styrénique. On peut par exemple utiliser un polymère contenant 0-60% de motifs dérivés du chlorure de vinylidène, 20-90% de motifs dérivés d'acrylonitrile et 0-50% de motifs dérivés d'un monomère acrylique ou styrénique, la somme des pourcentages (en poids) étant égale à 100%. Le monomère acrylique peut être le (méth)acrylate de méthyle ou d'éthyle. Le monomère styrénique peut être le styrène ou le α-méthyl-styrène.

Plus préférentiellement, les particules utilisées dans la présente invention sont des particules creuses d'un copolymère expansé de chlorure de vinylidène et d'acrylonitrile ou de chlorure de vinylidène, d'acrylonitrile et de méthacrylate de méthyle. Ces particules peuvent être sèches ou hydratées.

De façon préférentielle, la masse volumique de ces particules est choisie dans la gamme allant de 15 à 200 kg/m³ et mieux de 40 à 120 kg/m³, et encore mieux de 60 à 80 kg/m³.

Les particules utilisables dans l'invention sont par exemple les microsphères de terpolymère expansé de chlorure de vinylidène, d'acrylonitrile et de méthacrylate de méthyle, vendues sous la marque EXPANCEL par la société Nobel Casco et en particulier sous les références 551 DE 12 (granulométrie D(0,5) d'environ 12 µm et masse volumique d'environ 40 kg/m³), 551 DE 20 (granulométrie D(0,5) d'environ 15 à 25 µm et masse volumique d'environ 60 kg/m³), 551 DE 50 (granulométrie D(0,5) d'environ 40 µm), 461 DE 50 et 642 WE 50 de 50 µm de granulométrie D(0,5) environ, 551 DE 80 (granulométrie D(0,5) de 50 à 80 µm environ). On peut aussi utiliser des particules de ce même terpolymère expansé, ayant une granulométrie D(0,5) d'environ 18 µm et une masse volumique d'environ 60 à 80 kg/m³ (Expancel EL23) ou encore de granulométrie D(0,5) d'environ 34 µm et de masse volumique d'environ 20 kg/m³. On peut encore citer les particules EXPANCEL 551 DE 40 d42 (granulométrie D(0,5) d'environ 30 à 50 µm et masse volumique d'environ 42 kg/m³), 551 DE 80 d42 (granulométrie D(0,5) d'environ 50 à 80 µm et masse volumique d'environ 42 kg/m³), 461 DE 20 d70 (granulométrie D(0,5) d'environ 15 à 25 µm et masse volumique d'environ 70 kg/m³), 461 DE 40 d25 (granulométrie D(0,5) d'environ 35 à 55 µm et masse volumique d'environ 25 kg/m³), 461 DE 40 d60 (granulométrie D(0,5) d'environ 20 à 40 µm et masse volumique d'environ 60 kg/m³), 461 DET 40 d25 (granulométrie D(0,5) d'environ 35 à 55 µm et masse volumique d'environ 25 kg/m³), 051 DE 40 d60 (granulométrie D(0,5) d'environ 20 à 40 µm et masse volumique d'environ 60 kg/m³), 091 DE 40 d30 (granulométrie D(0,5) d'environ 35 à 55 µm et masse volumique d'environ 30 kg/m³), 091 DE 80 d30 (granulométrie D(0,5) d'environ 60 à 90 µm et masse volumique d'environ 30 kg/m³). On peut encore utiliser des particules de polymère de chlorure de vinylidène et d'acrylonitrile ou de chlorure de vinylidène, d'acrylonitrile et de méthacrylate de méthyle non expansé comme celles vendues sous la marque EXPANCEL avec la référence 551 DU 10 (granulométrie D(0,5) d'environ 10 µm) ou 461 DU 15 (granulométrie D(0,5) d'environ 15 µm).

Parmi les microsphères microporeuses utilisables dans la composition selon l'invention, on peut citer celles vendues par Dow Corning sous la dénomination 'POLYTRAP' qui sont formées de copolymères méthacrylate de lauryle/diméthacrylate d'éthylèneglycol; ou celles vendues par Seppic sous la dénomination 'MICROPEARL'.

Comme autres particules creuses polymériques utilisables dans l'invention, on peut encore citer les polymères et les copolymères obtenus à partir des esters ou acides, itaconique, citraconique, maléique, fumarique, de l'acétate ou lactate de vinyle. (Voir à cet effet le document JP-A-2-112304).

De préférence les agents structurants organiques sont des particules creuses.

Le ou les agents structurants organiques représentent de 1 à 10%, de préférence de 2 à 6%, de préférence encore de 2,5 à 5%, mieux de 3 à 4% en poids du poids total de la composition cosmétique.

Comme expliqué précédemment, la composition comprend en outre plusieurs agents absorbants choisis parmi les mélanges d'un ou plusieurs amidons modifiés et d'une ou plusieurs farines de bois.

Par agent absorbant, on entend tout composé susceptible de piéger rapidement une grande quantité d'eau. L'agent absorbant organique est donc généralement un composé hydrophile ou amphiphile.

Par agent absorbant au sens de la présente invention, on entend tout composé ayant une capacité statique d'absorption d'eau, à température ambiante (25°C), supérieure ou égale à 3 fois son poids.

De préférence, on choisit les agents absorbants parmi les composés ayant une capacité statique d'absorption d'eau supérieure ou égale à 5 fois son poids, et préférentiellement supérieure ou égale à 15 fois son poids.

Le test pour mesurer ladite capacité statique d'absorption d'eau est décrit avant les exemples.

Cet agent absorbant permet d'obtenir une composition sous forme de solide, notamment déformable, qui se délite aisément à l'aide d'un diluant qui est généralement de l'eau, froide ou chaude, mais qui peut également être de l'eau additionnée d'un ou plusieurs solvants polaires cosmétiquement acceptables, tels que les alcools ayant 2 à 20 atomes de carbone (isopropanol, éthanol notamment), le propylène glycol, ou encore de l'eau additionnée d'un ou plusieurs tensioactifs. On peut aussi utiliser des milieux aqueux plus complexes.

Par amidon modifié on entend au sens de la présente invention les amidons qui sont modifiés chimiquement par une ou plusieurs des réactions suivantes prégélatinisation, oxydation, réticulation, estérification, traitement thermique.

Les molécules d'amidons modifiés présents dans la présente invention peuvent provenir d'une source végétale telle que les céréales, les tubercules, les racines, les légumes et les fruits. Ainsi, le ou les amidons peuvent provenir d'une source végétale choisie parmi le maïs, les pois, la pomme de terre, la patate douce, la banane, l'orge, le blé, le riz, l'avoine, le sagou, le tapioca et le sorgo.

Le ou les amidons modifiés sont de préférence des amidons de pomme de terre.

On peut également utiliser les hydrolysats des amidons modifiés cités ci-dessus.

Les amidons modifiés se présentent généralement sous la forme d'une poudre blanche, insoluble dans l'eau froide, dont la taille des particules élémentaires va de 3 à 100 microns.

De manière plus particulière, les réactions de modification peuvent être réalisées de la façon suivante :
- prégélatinisation en faisant éclater les granules d'amidon (par exemple séchage et cuisson dans un tambour sécheur) ;
- oxydation par des oxydants forts conduisant à l'introduction de groupes carboxyle dans la molécule d'amidon et à la dépolymérisation de la molécule d'amidon (par exemple en traitant une solution aqueuse d'amidon par l'hypochlorite de sodium) ;
- réticulation par des agents fonctionnels capables de réagir avec les groupes hydroxyle des molécules d'amidon qui vont ainsi être liées entre elles (par exemple avec des groupes glycéryl et/ou phosphate) ;
- estérification en milieu alcalin pour le greffage de groupes fonctionnels, notamment acyl en C1-C6 (acétyl), hydroxyalkylés en C1-C6 (hydroxyéthyl, hydroxypropyl), carboxyméthyl, octénylsuccinique. On peut citer en particulier les amidons modifiés par le carboxyméthyl de sodium.

On peut notamment obtenir par réticulation avec des composés phosphorés des phosphates de monoamidon (du type Am-O-PO-(OX)2), des phosphates de diamidon (du type Am-O-PO-(OX)-O-Am) ou même de triamidon (du type Am-O-PO- (O-Am)2) ou leurs mélanges.

X désigne notamment les métaux alcalins (par exemple sodium ou potassium), les métaux alcalinoterreux (par exemple calcium, magnésium), les sels d'ammoniaque, les sels d'amines comme ceux de la monoéthanolamine, la diéthanolamine, la triéthanolamine, l'amino-3 propanediol-1,2, les sels ammoniums issus des aminoacides basiques tels que la lysine, l'arginine, la sarcosine, l'ornithine, la citrulline.

Les composés phosphorés peuvent être par exemple du tripolyphosphate de sodium, de l'orthophosphate de sodium, de l'oxychlorure de phosphore ou du trimétaphosphate de sodium.

Parmi les amidons modifiés susceptibles d'être utilisés, on peut citer les fécules de pomme de terre prégélatinisées quaternisées, les amidons de maïs prégélatinisés, les carboxyméthylamidons de pomme de terre réticulés, les phosphates de diamidon de manioc prégélatinisés et éventuellement hydroxypropylés, les phosphates de diamidon de pomme de terre prégélatinisés et éventuellement acétylés.

Parmi les produits commercialement disponibles, on peut citer les produits vendus par AVEBE sous les dénominations PREGEL ou PRIMOGEL, ou ceux vendus par NATIONAL STARCH sous la dénomination STRUCTURE ZEA.

Selon l'invention, on peut aussi utiliser des amidons amphotères, ces amidons amphotères contiennent un ou plusieurs groupements anioniques et un ou plusieurs groupements cationiques. Les groupements anioniques et cationiques peuvent être liés au même site réactif de la molécule d'amidon ou à des sites réactifs différents; de préférence ils sont liés au même site réactif. Les groupements anioniques peuvent être de type carboxylique, phosphate ou sulfate et de préférence carboxylique. Les groupements cationiques peuvent être de type amine primaire, secondaire, tertiaire ou quaternaire.

Les amidons amphotères sont notamment choisis parmi les composés de formules suivantes : 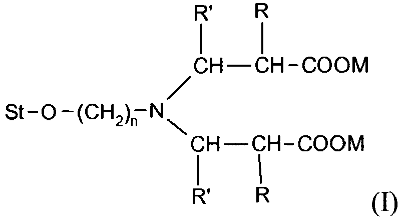 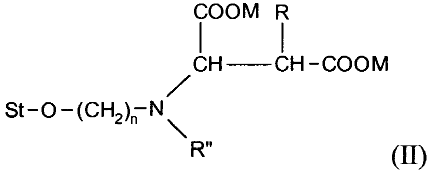 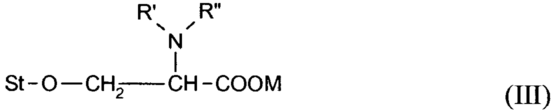 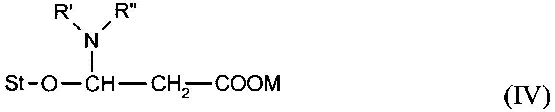 formules dans lesquelles :
St-O représente une molécule d'amidon,
R, identique ou différent, représente un atome d'hydrogène ou un radical méthyle,
R', identique ou différent, représente un atome d'hydrogène, un radical méthyle ou un groupement -COOH,
n est un entier égal à 2 ou 3,
M, identique ou différent, désigne un atome d'hydrogène, un métal alcalin ou alcalinoterreux tels que Na, K, Li, NH₄, un ammonium quaternaire ou une amine organique,
R" représente un atome d'hydrogène ou un radical alkyle ayant de 1 à 18 atomes de carbone.

Ces composés sont notamment décrits dans les brevets US 5,455,340 et US 4,017,460.

On utilise particulièrement les amidons de formules (I) ou (II). On utilise plus particulièrement les amidons modifiés par de l'acide 2-chloroéthyl aminodipropionique, c'est à dire les amidons de formule (I) ou (II) dans lesquelles R, R', R" et M représentent un atome d'hydrogène et n est égal à 2. On peut citer en particulier la fécule de pomme de terre modifiée par de l'acide 2-chloroéthyl aminodipropionique neutralisée à la soude, commercialisée sous la référence STRUCTURE SOLANACE par la société NATIONAL STARCH.

Les farines de bois peuvent être notamment la farine d'épicéa ou la farine de hêtre, de préférence la farine d'épicéa.

De préférence, la granulométrie moyenne des farines de bois est inférieure à 250 µm.

De tels produits sont notamment vendus par la Société Parisienne des Sciures sous la dénomination T140 (farine d'épicéa) ou H160/0 (farine de hêtre).

Ainsi, de préférence, l'agent absorbant est un mélange d'amidon modifié de pomme de terre et de farine d'épicéa.

Les agents absorbants représentent généralement de 3 à 50%, de préférence de 5 à 35%, mieux de 10 à 20%, en poids du poids total de la composition.

De préférence, le ou les amidons modifiés représentent de 3 à 25%, de préférence de 4 à 15% en poids du poids total de la composition.

De préférence, la ou les farines de bois représentent de 1 à 25%, mieux de 1,5 à 15% en poids du poids total de la composition.

Ainsi, dans un mode de réalisation particulièrement préféré, la composition selon l'invention comprend 4 à 12% en poids d'amidon modifié de pomme de terre, et 2 à 12% en poids de farine d'épicéa, par rapport au poids total de la composition.

Outre le ou les agents structurants organiques et les agents absorbants, la composition selon l'invention comprend un ou plusieurs agents tensioactifs.

Les tensioactifs qui sont de préférence anhydres, c'est-à-dire ne contenant pas d'eau ou contenant de l'eau à une teneur inférieure à 5% en poids, peuvent être choisis parmi les tensioactifs cationiques, anioniques, non ioniques ou amphotères.

Parmi les agents tensioactifs du type anionique pouvant être utilisés dans les compositions selon l'invention, on peut citer notamment les sels, en particulier les sels alcalins et notamment de sodium, les sels d'ammonium, les sels d'amines, les sels d'aminoalcools ou les sels de magnésium des composés suivants : les alkylsulfates, les alkyléthersulfates, les alkylamidoéthersulfates, les monoglycérides sulfates, les alkylglycérylsulfonates, les alkylsulfonates, les alkyl-phosphates, les alkylamidesulfonates, les alkylarylsulfonates, les α-oléfinesulfonates, les paraffines sulfonates, les alkylsulfosuccinates, les alkyléthersulfosuccinates, les alkylamidesulfosuccinates, les alkylsulfosuccinamates, les alkylsulfoacétates, les alkylétherphos-phates, les acyliséthionates, les N-acyltaurates, les N-acylaminoacides tels que les N-acylsarcosinates et les N-acylglutamates. On peut également citer comme agents tensioactifs anioniques pouvant être utilisés dans les compositions selon l'invention, les sels d'acides gras tels que les sels des acides undécénylique, oléique, ricinoléique, palmitique et stéarique, les acides d'huile de coprah ou d'huile de coprah hydrogénée et les acylhydroxyacides tels que les acyl-lactylates. On peut également utiliser des agents tensioactifs faiblement anioniques tels que les acides d'alkyl D-galactoside uroniques et leurs sels ainsi que les acides alkyléthers alkylamidoéthercarboxyliques polyoxyalkylénés ou leurs sels, le radical alkyle ou acyle de ces différents composés comportant de préférence de 8 à 22 atomes de carbone et les dérivés anioniques d'alkyle (C₈-C₂₂) polyglycosides (sulfate, sulfosuccinate, phosphate, iséthionate, éthercarboxylate, carbonate).

Parmi les agents tensioactifs du type amphotère on peut notamment citer les dérivés d'amines secondaires ou tertiaires aliphatiques, dans lesquels le radical aliphatique est une chaîne linéaire ou ramifiée comportant 8 à 22 atomes de carbone et contenant au moins un groupe anionique hydrosolubilisant tel que par exemple, un groupe carboxylate, sulfonate, sulfate, phosphate ou phosphonate. On peut encore citer parmi les agents tensioactifs de type amphotère ou zwittérionique les sulfobétaines, les alkylamidoalkylbétaïnes, les alkylamidoalkylsulfobétaines, les dérivés d'imidazolium tels que ceux d'amphocarboxyglycinate ou d'amphocarboxypropionate.

Parmi les agents tensioactifs de type non ionique des compositions selon l'invention, on peut citer notamment les dérivés polyéthoxylés polypropoxylés ou polyglycérolés des alcools ou des alphadiols ou des alkylphénols ou des acides gras, ayant une chaîne grasse comportant de 8 à 28 atomes de carbone, le nombre de groupes d'oxyde d'éthylène ou de propylène pouvant aller de 2 à 50 et celui de glycérol notamment de 2 à 30. On peut également citer les copolymères d'oxyde d'éthylène et de propylène, les condensats d'oxyde d'éthylène et de propylène sur des alcools gras, les amides gras polyéthoxylés ayant de préférence de 2 à 30 moles d'oxyde d'éthylène, les amides gras polyglycérolés comportant en moyenne 1 à 5 groupes glycérol, les diglycolamides polyglycérolés, les esters d'acides gras du sorbitan éventuellement oxyéthylénés, les esters d'acides gras du saccharose, les esters d'acides gras polyoxyalkylénés, les alkylpolyglycosides éventuellement oxyalkylénés, les esters d'alkylglucosides, les dérivés de N-alkylglucamine et de N-acyl-méthylglucamine, les aldobionamides et les oxydes d'amine.

Comme tensioactifs cationiques on peut citer en particulier (liste non limitative) : les sels d'amines grasses primaires, secondaires ou tertiaires, éventuellement polyoxyalkylénées ; les sels d'ammonium quaternaire tels que les chlorures ou les bromures de tétraalkylammonium, d'alkylamidoalkyltrialkylammonium, de trialkylbenzylammonium, de trialkylhydroxyalkyl-ammonium ou d'alkylpyridinium; les dérivés d'imidazoline.

On peut aussi utiliser à titre de tensioactifs cationiques les sels d'ammonium quaternaire contenant au moins une fonction ester tels que ceux de formule (V) suivante : 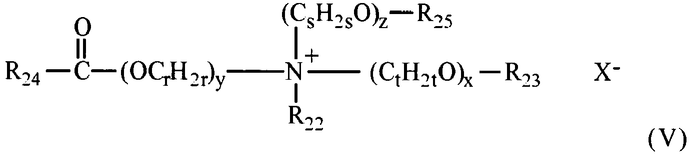 dans laquelle :
R₂₂ est choisi parmi les radicaux alkyles en C₁-C₆ et les radicaux hydroxyalkyles ou dihydroxyalkyles en C₁-C₆ ;
R₂₃ est choisi parmi :
   - le radical 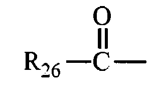
   - les radicaux R₂₇ hydrocarbonés en C₁-C₂₂, linéaires ou ramifiés, saturés ou insaturés,
   - l'atome d'hydrogène,
R₂₅ est choisi parmi :
   - le radical 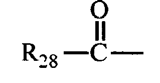
   - les radicaux R₂₉ hydrocarbonés en C₁-C₆, linéaires ou ramifiés, saturés ou insaturés,
   - l'atome d'hydrogène,
R₂₄, R₂₆ et R₂₈, identiques ou différents, sont choisis parmi les radicaux hydrocarbonés en C₇-C₂₁, linéaires ou ramifiés, saturés ou insaturés ;
r, s et t, identiques ou différents, sont des entiers valant de 2 à 6 ;
y est un entier valant de 1 à 10 ;
x et z, identiques ou différents, sont des entiers valant de 0 à 10 ;
X⁻ est un anion simple ou complexe, organique ou inorganique;
sous réserve que la somme x + y + z vaut de 1 à 15, que lorsque x vaut 0 alors R₂₃ désigne R₂₇ et que lorsque z vaut 0 alors R₂₅ désigne R₂₉.

Les radicaux alkyles R₂₂ peuvent être linéaires ou ramifiés et plus particulièrement linéaires.

De préférence R₂₂ désigne un radical méthyle, éthyle, hydroxyéthyle ou dihydroxypropyle, et plus particulièrement un radical méthyle ou éthyle.

Avantageusement, la somme x + y + z vaut de 1 à 10.

Lorsque R₂₃ est un radical R₂₇ hydrocarboné, il peut être long et avoir de 12 à 22 atomes de carbone, ou court et avoir de 1 à 3 atomes de carbone.

Lorsque R₂₅ est un radical R₂₉ hydrocarboné, il a de préférence 1 à 3 atomes de carbone.

Avantageusement, R₂₄, R₂₆ et R₂₈, identiques ou différents, sont choisis parmi les radicaux hydrocarbonés en C₁₁-C₂₁, linéaires ou ramifiés, saturés ou insaturés, et plus particulièrement parmi les radicaux alkyle et alcényle en C₁₁-C₂₁, linéaires ou ramifiés, saturés ou insaturés.

De préférence, x et z, identiques ou différents, valent 0 ou 1.

Avantageusement, y est égal à 1.

De préférence, r, s et t, identiques ou différents, valent 2 ou 3, et encore plus particulièrement sont égaux à 2.

L'anion est de préférence un halogénure (chlorure, bromure ou iodure) ou un alkylsulfate plus particulièrement méthylsulfate. On peut cependant utiliser le méthanesulfonate, le phosphate, le nitrate, le tosylate, un anion dérivé d'acide organique tel que l'acétate ou le lactate ou tout autre anion compatible avec l'ammonium à fonction ester.

L'anion X⁻ est encore plus particulièrement le chlorure ou le méthylsulfate.

Parmi les sels d'ammonium de formule (V), on utilise plus particulièrement les composés dans lesquels :
- R₂₂ désigne un radical méthyle ou éthyle,
- x et y sont égaux à 1 ;
- z est égal à 0 ou 1 ;
- r, s et t sont égaux à 2 ;
- R₂₃ est choisi parmi :
- le radical 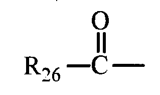
- les radicaux méthyle, éthyle ou hydrocarbonés en C₁₄-C₂₂,
- l'atome d'hydrogène ;
- R₂₅ est choisi parmi :
- le radical 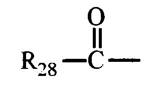
- l'atome d'hydrogène ;
- R₂₄, R₂₆ et R₂₈, identiques ou différents, sont choisis parmi les radicaux hydrocarbonés en C₁₃-C₁₇, linéaires ou ramifiés, saturés ou insaturés, et de préférence parmi les radicaux alkyles et alcényles en C₁₃-C₁₇, linéaires ou ramifiés, saturés ou insaturés.

Avantageusement, les radicaux hydrocarbonés sont linéaires.

On peut citer par exemple les composés de formule (V) tels que les sels (chlorure ou méthylsulfate notamment) de diacyloxyéthyldiméthylammonium, de diacyloxyéthyl-hydroxyéthyl-méthylammonium, de monoacyloxyéthyl-dihydroxyéthyl-méthylammonium, de triacyloxy éthyl-méthylammonium, de monoacyloxyéthyl-hydroxyéthyl-diméthyl ammonium et leurs mélanges. Les radicaux acyles ont de préférence 14 à 18 atomes de carbone et proviennent plus particulièrement d'une huile végétale comme l'huile de palme ou de tournesol. Lorsque le composé contient plusieurs radicaux acyles, ces derniers peuvent être identiques ou différents. On peut citer en particulier le méthosulfate de distéaroyléthyl hydroxyéthylammonium.

Ces produits sont obtenus, par exemple, par estérification directe de la triéthanolamine, de la triisopropanolamine, d'alkyldiéthanolamine ou d'alkyldiisopropanolamine éventuellement oxyalkylénées sur des acides gras ou sur des mélanges d'acides gras d'origine végétale ou animale, ou par transestérification de leurs esters méthyliques. Cette estérification est suivie d'une quaternisation à l'aide d'un agent d'alkylation tel qu'un halogénure d'alkyle (méthyle ou éthyle de préférence), un sulfate de dialkyle (méthyle ou éthyle de préférence), le méthane sulfonate de méthyle, le paratoluènesulfonate de méthyle, la chlorhydrine du glycol ou du glycérol.

La composition selon l'invention peut contenir un mélange de sels de mono-, di- et triester d'ammonium quaternaire avec une majorité en poids de sels de diester.

Comme mélange de sels d'ammonium, on peut utiliser par exemple le mélange contenant 15 à 30 % en poids de méthylsulfate d'acyloxyéthyl-dihydroxyéthyl-méthylammonium, 45 à 60% de méthyl sulfate de diacyloxyéthyl-hydroxyéthyl-méthylammonium et 15 à 30% de méthylsulfate de triacyloxyéthyl-méthylammonium, les radicaux acyles ayant de 14 à 18 atomes de carbone et provenant d'huile de palme éventuellement partiellement hydrogénée.

On peut aussi utiliser les sels d'ammonium contenant au moins une fonction ester décrits dans les brevets US-A-4874554 et US-A-4137180.

A titre d'exemples non limitatifs de tensioactifs convenant pour la réalisation des compositions selon l'invention, on peut citer le REWOPOL SB F 12 P dont l'actif est le laurylsulfosuccinate de sodium, le TEXAPON Z 95 P dont l'actif est le laurylsulfate de sodium, la GENAMIN KDMP dont l'actif est le chlorure de béhényltriméthylammonium, le DEHYQUART F 75 dont l'actif est le méthosulfate de dicétéaroyléthylhydroxyéthylméthylammonium et le TWEEN 21 dont l'actif est le monolaurate de sorbitane à 4 moles d'oxyde d'éthylène.

De façon avantageuse, le ou les tensioactifs sont anhydres et sont sous forme de poudre. Ils peuvent présenter une granulométrie allant de 5 à 50 µm et mieux de 10 à 20 µm.

Le ou les tensioactifs représentent généralement de 1 à 40%, de préférence de 2 à 20%, mieux de 4 à 20%, en poids du poids total de la composition.

Lorsque la composition est un shampooing, elle comprend généralement un ou plusieurs tensioactifs anioniques.

Lorsque la composition est un après-shampooing, elle contient de préférence un ou plusieurs tensioactifs cationiques à une teneur variant de 1 à 10%, mieux de 2 à 5%, en poids du poids total de la composition.

Comme expliqué précédemment, la composition selon l'invention comprend également une phase liquide anhydre.

La phase liquide anhydre peut comprendre un ou plusieurs composés choisis parmi les monoalcools, les polyols et les corps gras.

Les monoalcools peuvent être choisis parmi l'éthanol et l'isopropanol.

Les polyols peuvent être choisis parmi la glycérine et les glycols comme le propylèneglycol, l'hexylèneglycol ou les polyéthylèneglycols.

Les corps gras peuvent être choisis parmi les huiles, les cires et les silicones.

Les huiles utilisables dans la composition selon l'invention peuvent être choisies parmi les huiles minérales comme l'huile de paraffine et l'huile de vaseline ; les huiles d'origine animale comme le perhydrosqualène ; les huiles d'origine végétale telles que l'huile d'amande douce, d'avocat, de ricin, d'olive, de jojoba, de sésame, d'arachide, de pépins de raisin, de colza, de coprah, de noisettes, de palme, de noyau d'abricot, de calophyllum, de son, de riz, de germes de maïs, de germes de blé, de soja, de tournesol, de carthame, de passiflore, de seigle et le beurre de karité et sa fraction liquide ; les huiles de synthèse telles que les esters gras comme le myristate de butyle ou d'isopropyle, le stéarate d'hexadécyle, d'isopropyle, d'octyle ou d'isodécyle, l'oléate de décyle, le laurate d'héxyle, le dicaprylate de propylène glycol, les esters dérivés d'acide lanolique comme le lanolate d'isopropyle et le lanolate d'isocétyle, les isoparaffines, les acétylglycérides, les octanoates d'alcools et de polyalcools tels que ceux de glycol et de glycérol, les ricinoléates d'alcools et de polyalcools, les triglycérides d'acides gras ; les huiles de silicone telles que les cyclométhicones, les polydiméthylsiloxanes volatiles et/ou non volatiles ou encore les phényldiméthyl-siloxanes ; leurs mélanges.

Parmi les cires utilisables, on peut citer les cires d'abeille, les cires de lanoline et les cires d'insectes de Chine, les cires de Carnauba, de Candelilla, d'ouricurry, les cires de fibres de liège, les cires de canne à sucre, les cires du Japon, les cires de jojoba hydrogénées et les huiles hydrogénées telles que l'huile de tournesol hydrogénée, l'huile de ricin hydrogénée, l'huile de coprah hydrogénée et la lanoline hydrogénée ; les paraffines, les cires monocristallines, les cires de Montan et les ozokérites ; les cires de polyéthylène, les cires obtenues par la synthèse de Fischer-Tropsch, les copolymères cireux ainsi que leurs esters, et les cires de silicone telles que les polyalcoxy et polyalkylsiloxanes.

Les silicones éventuellement présentes dans la composition selon l'invention sont en particulier des polyorganosiloxanes qui peuvent se présenter sous forme de solutions aqueuses, c'est-à-dire solubilisées, ou éventuellement sous forme de dispersions ou microdispersions, ou d'émulsions aqueuses. Les polyorganosiloxanes peuvent également se présenter sous forme d'huiles, de cires, de résines ou de gommes.

Les organopolysiloxanes sont définis plus en détail dans l'ouvrage de Walter NOLL "Chemistry and Technology of Silicones" (1968) Academie Press.

Les silicones peuvent être volatiles ou non volatiles.

Lorsqu'elles sont volatiles, les silicones sont plus particulièrement choisies parmi celles possédant un point d'ébullition compris entre 60° C et 260° C, et plus particulièrement encore parmi :
(i) les silicones cycliques comportant de 3 à 7 atomes de silicium et de préférence 4 à 5.
   Il s'agit, par exemple, de l'octaméthylcyclotétrasiloxane commercialisé notamment sous le nom de "VOLATILE SILICONE 7207" par UNION CARBIDE ou "SILBIONE 70045 V 2" par RHONE POULENC, le décaméthylcyclopentasiloxane commercialisé sous le nom de "VOLATILE SILICONE 7158" par UNION CARBIDE, "SILBIONE 70045 V 5" par RHONE POULENC, ainsi que leurs mélanges.
   On peut également citer les cyclocopolymères du type diméthylsiloxanes/méthylalkylsiloxane, tel que la "SILICONE VOLATILE FZ 3109" commercialisée par la société UNION CARBIDE, de structure chimique : avec 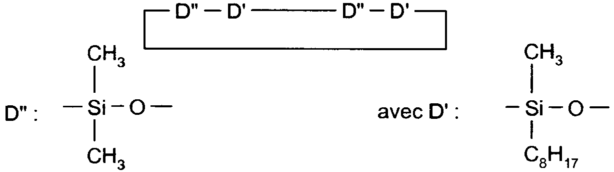
   On peut également citer les mélanges de silicones cycliques avec des composés organiques dérivés du silicium, tels que le mélange d'octaméthylcyclotétrasiloxane et de tétratriméthylsilylpentaérythritol (50/50) et le mélange d'octaméthylcyclotétrasiloxane et d'oxy-1,1'(hexa-2,2,2',2',3,3'-triméthylsilyloxy) bis-néopentane ;
(ii) les silicones volatiles linéaires ayant 2 à 9 atomes de silicium et possédant une viscosité inférieure ou égale à 5.10⁻⁶m²/s à 25° C. Il s'agit, par exemple, du décaméthyltétrasiloxane commercialisé notamment sous la dénomination "SH 200" par la société TORAY SILICONE. Des silicones entrant dans cette classe sont également décrites dans l'article publié dans Cosmetics and toiletries, Vol. 91, Jan. 76, P. 27-32 - TODD & BYERS "Volatile Silicone fluids for cosmetics".

On utilise de préférence des silicones non volatiles et plus particulièrement des polyalkylsiloxanes, des polyarylsiloxanes, des polyalkylarylsiloxanes, des gommes et des résines de silicones, des polyorganosiloxanes modifiés par des groupements organofonctionnels ainsi que leurs mélanges.

Ces silicones sont plus particulièrement choisies parmi les polyalkylsiloxanes parmi lesquels on peut citer principalement les polydiméthylsiloxanes à groupements terminaux triméthylsilyle (Diméthicone selon la dénomination CTFA) ayant une viscosité de 5.10⁻⁶ à 2,5 m²/s à 25°C et de préférence 1.10⁻⁵ à 1 m²/s. La viscosité des silicones est par exemple mesurée à 25°C selon la norme ASTM 445 Appendice C.

Parmi ces polyalkylsiloxanes, on peut citer à titre non limitatif les produits commerciaux suivants :
- les huiles SILBIONE des séries 47 et 70 047 ou les huiles MIRASIL commercialisées par RHONE POULENC telles que par exemple l'huile 70 047 V 500 000 ;
- les huiles de la série MIRASIL commercialisées par la société RHONE POULENC ;
- les huiles de la série 200 de la société DOW CORNING telles que plus particulièrement la DC200 de viscosité 60 000 Cst ;
- les huiles VISCASIL de GENERAL ELECTRIC et certaines huiles des séries SF (SF 96, SF 18) de GENERAL ELECTRIC.

On peut également citer les polydiméthylsiloxanes à groupements terminaux diméthylsilanol (Dimethiconol selon la dénomination CTFA) tels que les huiles de la série 48 de la société RHONE POULENC.

On peut également citer les polydiméthylsiloxanes à groupements aminoéthyl aminopropyl et alpha-oméga silanols.

Dans cette classe de polyalkylsiloxanes, on peut également citer les produits commercialisés sous les dénominations "ABIL WAX 9800 et 9801" par la société GOLDSCHMIDT qui sont des polyalkyl (C₁-C₂₀) siloxanes.

Les polyalkylarylsiloxanes sont particulièrement choisis parmi les polydiméthyl méthylphénylsiloxanes, les polydiméthyl diphénylsiloxanes linéaires et/ou ramifiés de viscosité de 1.10⁻⁵ à 5.10⁻²m²/s à 25°C.

Parmi ces polyalkylarylsiloxanes on peut citer à titre d'exemple les produits commercialisés sous les dénominations suivantes :
- les huiles SILBIONE de la série 70 641 de RHONE POULENC ;
- les huiles des séries RHODORSIL 70 633 et 763 de RHONE POULENC ;
- l'huile DOW CORNING 556 COSMETIC GRAD FLUID de DOW CORNING ;
- les silicones de la série PK de BAYER comme le produit PK20 ;
- les silicones des séries PN, PH de BAYER comme les produits PN1000 et PH1000 ;
- certaines huiles des séries SF de GENERAL ELECTRIC telles que SF 1023, SF 1154, SF 1250, SF 1265.

Les gommes de silicone pouvant être présentes dans la composition selon l'invention sont notamment des polydiorganosiloxanes ayant des masses moléculaires moyennes en nombre élevées comprises entre 200 000 et 1 000 000 utilisés seuls ou en mélange dans un solvant. Ce solvant peut être choisi parmi les silicones volatiles, les huiles polydiméthylsiloxanes (PDMS), les huiles poly-phénylméthylsiloxanes (PPMS), les isoparaffines, les polyisobutylènes, le chlorure de méthylène, le pentane, le dodécane, le tridécanes ou leurs mélanges.

On peut plus particulièrement citer les produits suivants :
- les gommes de polydiméthylsiloxane,
- les gommes polydiméthylsiloxanes/méthylvinylsiloxane,
- les gommes de polydiméthylsiloxane/diphénylsiloxane,
- les gommes de polydiméthylsiloxane/phénylméthylsiloxane,
- les gommes de polydiméthylsiloxane/diphénylsiloxane/méthylvinylsiloxane.

Des produits plus particulièrement utilisables sont les mélanges suivants:
- les mélanges formés à partir d'un polydiméthylsiloxane hydroxylé en bout de chaîne (dénommé diméthiconol selon la nomenclature du dictionnaire CTFA) et d'un poly-diméthylsiloxane cyclique (dénommé cyclométhicone selon la nomenclature du dictionnaire CTFA) tel que le produit Q2 1401 commercialisé par la société DOW CORNING ;
- les mélanges formés à partir d'une gomme polydiméthylsiloxane avec une silicone cyclique tel que le produit SF 1214 Silicone Fluid de la société GENERAL ELECTRIC, ce produit est une gomme SF 30 correspondant à une diméthicone, ayant un poids moléculaire moyen en nombre de 500 000 solubilisée dans l'huile SF 1202 Silicone Fluid correspondant au décaméthylcyclopentasiloxane ;
- les mélanges de deux PDMS de viscosités différentes, et plus particulièrement d'une gomme PDMS et d'une huile PDMS, tels que le produit SF 1236 de la société GENERAL ELECTRIC. Le produit SF 1236 est le mélange d'une gomme SE 30 définie ci-dessus ayant une viscosité de 20 m²/s et d'une huile SF 96 d'une viscosité de 5.10⁻⁶m²/s. Ce produit comporte de préférence 15 % de gomme SE 30 et 85 % d'une huile SF 96.

Les résines d'organopolysiloxanes éventuellement présentes dans la composition selon l'invention sont des systèmes siloxaniques réticulés renfermant les unités : R₂SiO_{2/2}, R₃SiO_{1/2}, RSiO_{3/2} et SiO_{4/2} dans lesquelles R représente un groupement hydrocarboné possédant 1 à 16 atomes de carbone ou un groupement phényle. Parmi ces produits, ceux particulièrement préférés sont ceux dans lesquels R désigne un radical alkyle inférieur en C₁-C₄, plus particulièrement méthyle, ou un radical phényle.

On peut citer parmi ces résines le produit commercialisé sous la dénomination "DOW CORNING 593" ou ceux commercialisés sous les dénominations "SILICONE FLUID SS 4230 et SS 4267" par la société GENERAL ELECTRIC et qui sont des silicones de structure diméthyl/triméthyl siloxane.

On peut également citer les résines du type triméthylsiloxysilicate commercialisées notamment sous les dénominations X22-4914, X21-5034 et X21-5037 par la société SHIN-ETSU.

Les silicones organomodifiées éventuellement présentes dans la composition selon l'invention sont des silicones telles que définies précédemment et comportant dans leur structure un ou plusieurs groupements organofonctionnels fixés par l'intermédiaire d'un radical hydrocarboné.

Parmi les silicones organomodifiées, on peut citer les polyorganosiloxanes comportant :
- des groupements polyéthylèneoxy et/ou polypropylèneoxy comportant éventuellement des groupements alkyle en C₆-C₂₄ tels que les produits dénommés diméthicone copolyol commercialisé par la société DOW CORNING sous la dénomination DC 1248 ou les huiles SILWET L 722, L 7500, L 77, L 711 de la société UNION CARBIDE et l'alkyl (C₁₂) méthicone copolyol commercialisée par la société DOW CORNING sous la dénomination Q2 5200 ;
- des groupements thiols comme les produits commercialisés sous les dénominations "GP 72 A" et "GP 71" de GENESEE ;
- des groupements alcoxylés comme le produit commercialisé sous la dénomination "SILICONE COPOLYMER F-755" par SWS SILICONES et ABIL WAX 2428, 2434 et 2440 par la société GOLDSCHMIDT ;
- des groupements hydroxylés comme les polyorganosiloxanes à fonction hydroxyalkyle décrits dans la demande de brevet français FR-A-85 16334 ;
- des groupements acyloxyalkyle tels que par exemple les polyorganosiloxanes décrits dans le brevet US-A-4957732 ;
- des groupements anioniques du type carboxylique comme par exemple dans les produits décrits dans le brevet EP 186 507 de la société CHISSO CORPORATION, ou de type alkylcarboxyliques comme ceux présents dans le produit X-22-3701E de la société SHIN-ETSU ; 2-hydroxyalkylsulfonate ; 2-hydroxyalkylthiosulfate tels que les produits commercialisés par la société GOLDSCHMIDT sous les dénominations "ABIL S201" et "ABIL S255" ;
- des groupements hydroxyacylamino, comme les polyorganosiloxanes décrits dans la demande EP 342 834. On peut citer par exemple le produit Q2-8413 de la société DOW CORNING.

Parmi les silicones organomodifiées, on peut encore citer les silicones aminées.

Par silicone aminée, on entend toute silicone comportant au moins une fonction amine primaire, secondaire, tertiaire ou un groupement ammonium quaternaire.

Les silicones aminées éventuellement utilisées dans la composition cosmétique selon la présente invention sont choisies parmi :
(a) les composés répondant à la formule (VI) suivante :

   (R¹)ₐ(T)₃₋ₐ-Si[OSi(T)₂]ₙ-[OSi(T)b(R¹)_{2-b}]ₘ-OSi(T)₃₋ₐ-(R¹)ₐ (VI)

   dans laquelle,
   T est un atome d'hydrogène, ou un radical phényle, hydroxyle (-OH), ou alkyle en C₁-C₈, et de préférence méthyle ou alcoxy en C₁-C₈, de préférence méthoxy,
   a désigne le nombre 0 ou un nombre entier de 1 à 3, et de préférence 0,
   b désigne 0 ou 1, et en particulier 1,
   m et n sont des nombres tels que la somme (n + m) peut varier notamment de 1 à 2 000 et en particulier de 50 à 150, n pouvant désigner un nombre de 0 à 1 999 et notamment de 49 à 149 et m pouvant désigner un nombre de 1 à 2 000, et notamment de 1 à 10 ;
   R₁ est un radical monovalent de formule -C_{q}H_{2q}L dans laquelle q est un nombre de 2 à 8 et L est un groupement aminé éventuellement quaternisé choisi parmi les groupements :

      -N(R²)-CH₂-CH₂-N(R²)₂ ;

      -N(R²)₂ ; -N⁺(R²)₃ Q⁻ ;

      -N⁺(R²)(H)₂ Q⁻ ;

      -N⁺(R²)₂HQ⁻ ;

      -N(R²)-CH₂-CH₂-N⁺(R²)(H)₂ Q⁻,
   dans lesquels R² peut désigner un atome d'hydrogène, un phényle, un benzyle, ou un radical hydrocarboné saturé monovalent, par exemple un radical alkyle en C₁-C₂₀, et Q- représente un ion halogénure tel que par exemple fluorure, chlorure, bromure ou iodure.

En particulier, les silicones aminées correspondant à la définition de la formule (VI) sont choisies parmi les composés correspondant à la formule suivante : 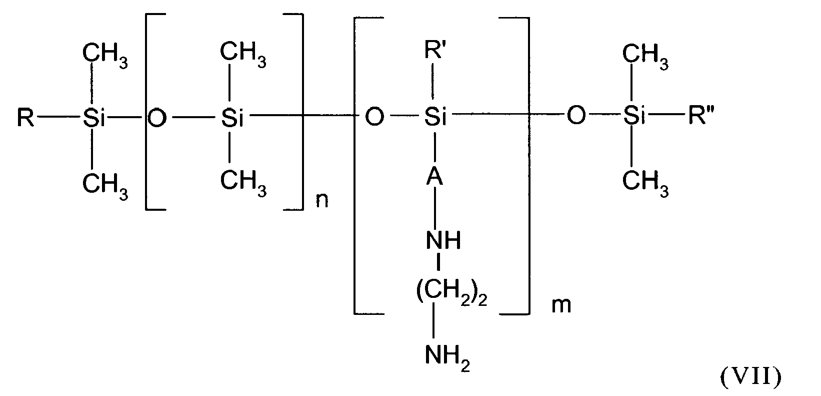 dans laquelle R, R', R", identiques ou différents, désignent un radical alkyle en C₁-C₄, de préférence CH₃ ; un radical alcoxy en C₁-C₄, de préférence méthoxy ; ou OH ; A représente un radical alkylène, linéaire ou ramifié, en C₃-C₈, de préférence en C₃-C₆; m et n sont des nombres entiers dépendant du poids moléculaire et dont la somme est comprise entre 1 et 2000.

Selon une première possibilité, R, R', R", identiques ou différents, représentent un radical alkyle en C₁-C₄ ou hydroxyle, A représente un radical alkylène en C₃ et m et n sont tels que la masse moléculaire moyenne en poids du composé est comprise entre 5 000 et 500 000 environ. Les composés de ce type sont dénommés dans le dictionnaire CTFA, "amodiméthicone".

Selon une deuxième possibilité, R, R', R", identiques ou différents, représentent un radical alcoxy en C₁-C₄ ou hydroxyle, l'un au moins des radicaux R ou R" est un radical alcoxy et A représente un radical alkylène en C₃. Le rapport molaire hydroxy / alcoxy est de préférence compris entre 0,2/1 et 0,4/1 et avantageusement égal à 0,3/1. Par ailleurs, m et n sont tels que la masse moléculaire moyenne en poids du composé est comprise entre 2000 et 10⁶. Plus particulièrement, n est compris entre 0 et 999 et m est compris entre 1 et 1000, la somme de n et m étant comprise entre 1 et 1000.

Dans cette catégorie de composés, on peut citer entre autres, le produit Belsil®ADM 652, commercialisée par Wacker.

Selon une troisième possibilité, R, R", différents, représentent un radical alcoxy en C₁-C₄ ou hydroxyle, l'un au moins des radicaux R, R" est un radical alcoxy, R' représente un radical méthyle et A représente un radical alkylène en C₃. Le rapport molaire hydroxy / alcoxy est de préférence compris entre 1/0,8 et 1/1,1, et avantageusement est égal à 1/0,95. Par ailleurs, m et n sont tels que la masse moléculaire moyenne en poids du composé est comprise entre 2000 et 200000. Plus particulièrement, n est compris entre 0 et 999 et m est compris entre 1 et 1000, la somme de n et m étant comprise entre 1 et 1000.

Plus particulièrement, on peut citer le produit FluidWR® 1300, commercialisé par Wacker.

Notons que la masse moléculaire de ces silicones est déterminée par chromatographie par perméation de gel (température ambiante, étalon polystyrène ; colonnes µ styragem ; éluant THF ; débit de 1 mm/m ; on injecte 200 µl d'une solution à 0,5 % en poids de silicone dans le THF et l'on effectue la détection par réfractométrie et UV-métrie).

Un produit correspondant à la définition de la formule (VI) est en particulier le polymère dénommé dans le dictionnaire CTFA "triméthylsilylamodiméthicone", répondant à la formule (VIII) suivante: 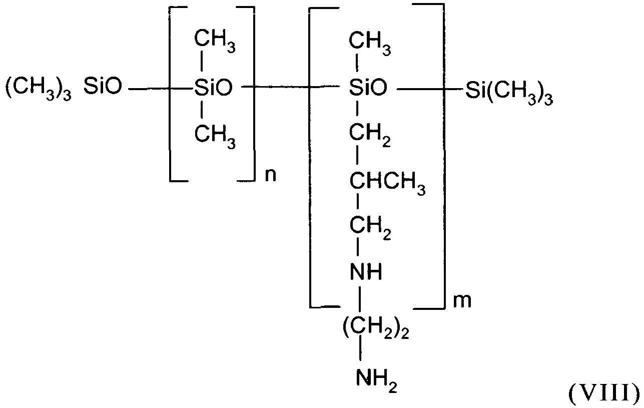 dans laquelle n et m ont les significations données ci-dessus conformément à la formule (VI).

De tels composés sont décrits par exemple dans EP 95238; un composé de formule (VIII) est par exemple vendu sous la dénomination Q2-8220 par la société OSI.
(b) les composés répondant à la formule (IX) suivante : 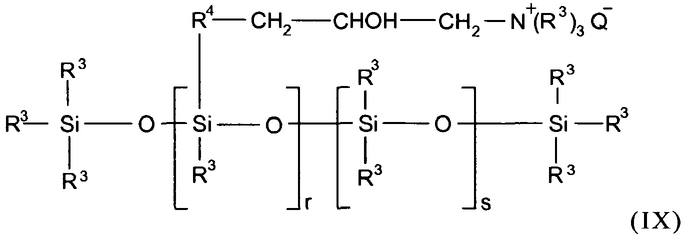 dans laquelle,
   R³ représente un radical hydrocarboné monovalent en C₁-C₁₈, et en particulier un radical alkyle en C₁-C₁₈, ou alcényle en C₂-C₁₈, par exemple méthyle ;
   R⁴ représente un radical hydrocarboné divalent, notamment un radical alkylène en C₁-C₁₈ ou un radical alkylèneoxy divalent en C₁-C₁₈, par exemple en C₁-C₈ ;
   Q⁻ est un ion halogénure, notamment chlorure ;
   r représente une valeur statistique moyenne de 2 à 20 et en particulier de 2 à 8 ;
   s représente une valeur statistique moyenne de 20 à 200 et en particulier de 20 à 50.

De tels composés sont décrits plus particulièrement dans le brevet US 4185087.

Un composé entrant dans cette classe est celui vendu par la Société Union Carbide sous la dénomination "Ucar Silicone ALE 56".
c) les silicones ammonium quaternaire de formule (X) : 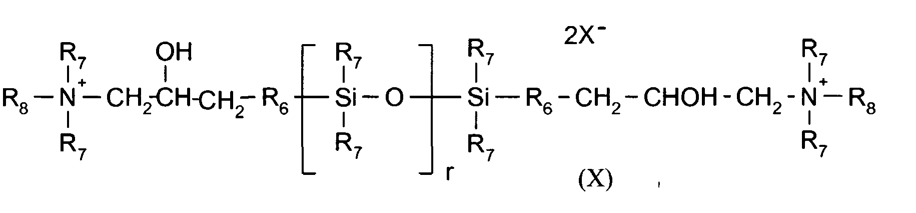 dans laquelle :
   R₇, identiques ou différents, représentent un radical hydrocarboné monovalent ayant de 1 à 18 atomes de carbone, et en particulier un radical alkyle en C₁-C₁₈, un radical alcényle en C₂-C₁₈ ou un cycle comprenant 5 ou 6 atomes de carbone, par exemple méthyle ;
   R₆ représente un radical hydrocarboné divalent, notamment un radical alkylène en C₁-C₁₈ ou un radical alkylèneoxy divalent en C₁-C₁₈, par exemple en C₁-C₈ relié au Si par une liaison SiC ;
   R₈, identiques ou différents, représentent un atome d'hydrogène, un radical hydrocarboné monovalent ayant de 1 à 18 atomes de carbone, et en particulier un radical alkyle en C₁-C₁₈, un radical alcényle en C₂-C₁₈ , un radical -R₆-NHCOR₇ ;
   X- est un anion tel qu'un ion halogénure, notamment chlorure ou un sel d'acide organique (acétate ...);
   r représente une valeur statistique moyenne de 2 à 200 et en particulier de 5 à 100 ;

Ces silicones sont par exemple décrites dans la demande EP-A-0530974.
d) les silicones aminées de formule (XI) : 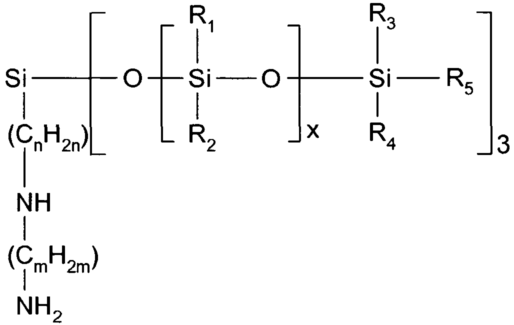 dans laquelle :
   - R₁, R₂, R₃ et R₄, identiques ou différents, désignent un radical alkyle en C₁-C₄ ou un groupement phényle,
   - R₅ désigne un radical alkyle en C₁-C₄ ou un groupement hydroxyle,
   - n est un entier variant de 1 à 5,
   - m est un entier variant de 1 à 5,
et dans laquelle x est choisi de manière telle que l'indice d'amine soit compris entre 0,01 et 1 meq/g.

Les silicones particulièrement préférées sont les polydiméthylsiloxanes, les diméthicones et les amodiméthicones.

Lorsque ces composés sont mis en oeuvre, une forme de réalisation particulièrement intéressante est leur utilisation conjointe avec des agents de surface cationiques et/ou non ioniques.

A titre exemple, on peut utiliser le produit vendu sous la dénomination "Emulsion Cationique DC 939" par la Société Dow Corning, qui comprend, outre l'amodiméthicone, un agent de surface cationique qui est le chlorure de triméthylcétylammonium et un agent de surface non ionique de formule : C₁₃H₂₇-(OC₂H₄)₁₂-OH, connu sous la dénomination CTFA "tridéceth-12".

Un autre produit commercial utilisable selon l'invention est le produit vendu sous la dénomination "Dow Corning Q2 7224" par la Société Dow Corning, comportant en association le triméthylsilylamodiméthicone de formule (VIII) décrite ci-dessus, un agent de surface non ionique de formule : C₈H₁₇-C₆H₄-(OCH₂CH2)₄₀-OH, connu sous la dénomination CTFA "octoxynol-40", un second agent de surface non ionique de formule: C₁₂H₂₅-(OCH₂-CH₂)₆-OH, connu sous la dénomination CTFA "isolaureth-6", et du propylèneglycol.

La ou les silicones représentent généralement 0,1 à 20%, de préférence de 0,1 à 10%, en poids du poids total de la composition.

La phase liquide anhydre représente de préférence au moins 50%, de préférence de 50 à 95%, en poids du poids total de la composition.

La composition selon l'invention peut en outre comprendre tout additif susceptible d'être utilisé dans le domaine d'application considéré.

En particulier, elle peut comprendre des polymères cationiques, des charges, des agents coiffants, des agents chauffants, des actifs cosmétiques ou dermatologiques, des parfums, des filtres UV, des conservateurs, des agents régulateurs de pH, des agents régulateurs de viscosité, des séquestrants, des agents anti-radicaux libres, des hydratants, des agents réducteurs ou antioxydants, des agents oxydants, des colorants, des agents conditionneurs, et des vitamines.

Parmi les charges, on peut citer les particules gommantes, les agents améliorant le délitement, les agents diminuant la fermeté de la composition.

Par agent chauffant, on entend un agent susceptible de dégager de la chaleur, lors de l'hydratation de la composition. Cet agent est notamment choisi parmi les polyols ayant au moins 2 groupes hydroxyle et au moins 3 atomes de carbone, comme notamment la glycérine, la diglycérine, le propylène glycol, le butylène glycol, un polyéthylène glycol de poids moléculaire inférieur à 600 comme le PEG 400 vendu par la société BASF sous le nom Lutrol E400, les sucres tels que le sorbitol, leurs mélanges. Ce type d'agent présente la particularité de réagir avec l'eau selon un processus exothermique. Pour que ce processus exothermique ait lieu, il est souhaitable que la composition soit exempte d'eau.

Comme actif cosmétique ou dermatologique utilisable dans la composition selon l'invention, on peut citer des antibactériens ou antifongiques comme l'octopirox et le triclosan, des agents kératolytiques comme l'acide salicylique, des huiles essentielles, des vitamines lipophiles, des agents anti-pelliculaires, des agents antichute.

La composition selon l'invention peut être une composition de shampooing, d'avant-shampooing, d'après-shampooing, de mise en forme des cheveux, de mise en forme permanente ou de défrisage, de coloration ou de décoloration, une composition pour le conditionnement des cheveux ou une composition moussante pour la peau (corps et/ou visage).

La composition selon l'invention est généralement rincée.

La composition selon l'invention peut se présenter sous forme de bâton, de crayon, de stick, de pain, voire de pâte, et constituer en elle-même un nouveau type de produit cosmétique ou pharmaceutique.

La composition selon l'invention peut se présenter sous forme d'un solide déformable.

On entend par 'déformable' le fait que la composition se présente sous une forme solide, sèche, malléable, ressemblant à de la guimauve (voir le document US-A-3 682 659 pour la consistance de la guimauve).

La composition selon l'invention est avantageusement appliquée sur des matières kératiniques mouillées. Elle peut cependant être utilisée sur des matières kératiniques sèches avec ajout d'une fraction d'eau après application de la composition.

En particulier, l'invention a encore pour objet l'utilisation d'une composition selon l'invention pour le nettoyage et/ou le conditionnement des matières kératiniques (peau et/ou cheveux).

La composition selon l'invention peut être préparée par tout moyen connu de l'homme du métier, et en particulier par simple mélange des différents constituants et moulage dans un moule adéquat. Elle peut encore être par mélange suivi de malaxage et extrusion dans un extrudeur, de préférence un extrudeur bi-vis, notamment un extrudeur bi-vis telle que celles décrites dans les brevets EP605284 ou FR2715306, et dans laquelle les deux vis tournent dans le même sens.

La masse extrudée sort de la filière d'extrusion sous la forme de boudins de diamètre donné selon la filière utilisée, pouvant être ensuite découpés et mis en forme, notamment, de bâton ou de pain solide. D'autres formes peuvent bien entendu être réalisées en choisissant des filières appropriées et des dispositifs de mise en forme des produits finaux adaptés à la forme recherchée.

La masse extrudée peut également être déshydratée et/ou broyée et/ou compactée après son obtention.

Le procédé d'extrusion peut être effectué à chaud, à température ambiante ou en présence d'un système de réfrigération. De préférence la totalité du procédé d'extrusion est réalisé à température ambiante, de l'ordre de 20-25°C, ou à froid, ce qui permet l'utilisation de matières premières sensibles à la chaleur, du type vitamines ou huiles volatiles.

D'autre part, ceci permet d'introduire les matières premières sensibles à la chaleur dans n'importe quelle zone de l'extrudeur (en tête, au milieu ou en final) puisque aucune détérioration due à la chaleur n'est à craindre. Ceci est en particulier avantageux pour l'introduction des agents structurant du type EXPANCEL.

Il est également possible d'effectuer une partie de l'extrusion sous gaz inerte (azote par exemple), ce qui peut être avantageux lorsque l'on emploie des produits oxydables.

L'extrusion étant généralement effectuée à température ambiante, la matrice formant la composition n'est pas une matrice d'un réseau expansé.

L'invention est illustrée par les exemples suivants.

### Test de mesure de la capacité statique d'absorption d'eau

A température ambiante, on dispose dans un bécher le composé à tester en une quantité de x grammes; on ajoute de l'eau en une quantité de 3x grammes. On laisse reposer, sans agiter, pendant 1 minute.

S'il ne reste plus d'eau libre (eau surnageante) après ladite minute, le composé peut être considéré comme un agent absorbant au sens de l'invention.

### Exemple 1

On formule une composition moussante lavante selon l'invention dont la formulation est la suivante (en poids par rapport au poids total de la composition) :

| | |
|---|---|
| - Microsphères de copolymère chlorure de vinylidène/acrylonitrile/méthacrylate de méthyle creuses expansées avec isobutane (Expancel 551 DE 40 d42 de la société Expancel): | 2,55% |
| - Mélange 96/4 de sel de sodium de carboxyméthylamidon de pomme de terre et d'éthanol (Primojel de la société DMV International): | 5,95% |
| - farine d'épicéa (farine de bois F140 de la société SPPS) : | 11,9% |
| - propylène glycol : | 20,4% |
| - glycérine : | 40,8% |
| - Laurylsulfate de sodium: | 17,85% |

### Exemple 2

On formule une composition de conditionnement des cheveux selon l'invention dont la formulation est la suivante (en poids par rapport au poids total de la composition) :

| | |
|---|---|
| - Microsphères de copolymère chlorure de vinylidène/acrylonitrile/méthacrylate de méthyle creuses expansées avec isobutane (Expancel 551 DE 40 d42 de la société Expancel): | 3,8 % |
| - Mélange 96/4 de sel de sodium de carboxyméthylamidon de pomme de terre et d'éthanol (Primojel de la société DMV International): | 12,3% |
| - farine d'épicéa (farine de bois F140 de la société SPPS) : | 5,3% |
| - propylèneglycol: | 73% |
| - Chlorure de béhényltriméthylammonium (Genamin KDMP de Clariant): | 3,2% |
| - alcool cétylstéarylique : | 1,6% |
| - mélange myristate/palmitate, stéarate de myristyle/cétyle/stéaryle : | 0,8% |

### Exemple 3

On formule une composition de conditionnement des cheveux selon l'invention dont la formulation est la suivante (en poids par rapport au poids total de la composition) :

| | |
|---|---|
| - Microsphères de copolymère chlorure de vinylidène/acrylonitrile/méthacrylate de méthyle creuses expansées avec isobutane (Expancel 551 DE 40 d42 de la société Expancel): | 6,4 % |
| - Mélange 96/4 de sel de sodium de carboxyméthylamidon de pomme de terre et d'éthanol (Primojel de la société DMV International): | 8,0% |
| - farine d'épicéa (farine de bois F140 de la société SPPS) : | 3,4% |
| - propylèneglycol: | 73% |
| - Chlorure de béhényltriméthylammonium (Genamin KDMP de Clariant): | 3,3% |
| - alcool cétylstéarylique : | 3,3% |
| - colorants, parfum | qs |
| - propylèneglycol: | qsp 100 |
| % soit environ 75%) | |

### Exemple 4

On formule différentes compositions de shampooing anhydre selon l'invention. Les formulations sont données dans le tableau 1 (en pourcentage en poids par rapport au poids total de la composition).

**Tableau 1**

| | **1** | **2** | **3** | **4** | **5** |
|---|---|---|---|---|---|
| Microsphères de copolymère chlorure de vinylidène/ acrylonitrile/ méthacrylate de méthyle creuses expansées avec isobutane (Expancel 551 DE 40 d42 de la société Expancel) | 3,3 | 2,6 | 3,3 | 2,6 | 2 |
| Colorant sel di-sodique de tartrazine : Yellow 5 (LCW) | 0,004 | 0,004 | 0,004 | 0,004 | 0,004 |
| Colorant sel di-sodique de bleu brillant : Blue 1 (LCW) | 0,005 | 0,005 | 0,005 | 0,005 | 0,005 |
| Farine d'épicéa (F140 de SPPS) | 10,2 | 11,9 | 10,3 | 11,9 | 12 |
| Parfum | 0,5 | 0,5 | 0,5 | 0,5 | 0,5 |
| Mélange 96/4 de sel de sodium de carboxyméthyl amidon de pomme de terre et d'éthanol (Primojel de la société DMV International) | 5,1 | 5,9 | 5,1 | 5,9 | 6 |
| Disodium lauryl sulfosuccinate: Rewopol SB F 12 P (Degussa) | | | | 17,9 | 17,9 |
| Lauryl sulfate de sodium en poudre : texapon Z 95 P (Cognis) | 15,3 | 17,9 | 15,3 | | |
| Polyéthylène glycol 400 | Qsp 100 (environ 65,6) | | | | |
| Glycérine | | 40,8 | 43,7 | 40,8 | 41,1 |
| Propylène glycol | | Qsp 100 (environ 20,4) | Qsp 100 (environ 21,8) | Qsp 100 (environ 20,4) | Qsp 100 (envi -ron 20,5) |

Toutes ces compositions ont une texture agréable et souple plus ou moins ferme mais se délitant facilement au contact de l'eau en générant un effet chaleur.

### Exemple 5

On formule différentes compositions de soin à rincer anhydre selon l'invention. Les formulations sont données dans le tableau 2 (en poids par rapport au poids total de la composition).

**Tableau 2**

| | **6** | **7** | **8** | **9** | **10** | **11** |
|---|---|---|---|---|---|---|
| Microsphères de copolymère chlorure de vinylidène/ acrylonitrile/ méthacrylate de méthyle creuses expansées avec isobutane (Expancel 551 DE 40 d42 de la société Expancel) | 4,35 | 3,5 | 4,2 | 3,4 | 3,8 | 3,5 |
| Alcool cétylstéarylique: Lanette O Or (COGNIS) | | | | | 1,6 | 1,2 |
| mélange myristate/ palmitate/ stéarate de myristyle/ cétyle/ stéaryle : Miraceti (Laserson) | | | | | 0,8 | 0,6 |
| Farine d'épicéa (F140 de SPPS) | 1,9 | 5,3 | 2,9 | 7,8 | 5,3 | 10,4 |
| Mélange 96/4 de sel de sodium de carboxyméthylamidon de | 4,45 | 12,4 | 6,7 | 18,4 | 12,3 | 24,2 |
| pomme de terre et d'éthanol (Primojel de la société DMV International) | | | | | | |
| Chlorure de béhényl triméthyl ammonium : Genamin KDMP (Clariant) | 4,0 | 3,5 | | | 3,2 | 2,5 |
| Méthosulfate de dicétéaroyléthyl hydroxyéthyl méthyl ammonium/ alcool cétéarylique : Dehyquart F 75 (Cognis) | | | 3,9 | 3,2 | | |
| Propylène glycol | QSp 100 (envi -ron 85,3) | Qsp 100 (envi -ron 75,3) | Qsp 100 (envi -ron 82,3) | Qsp 100 (envi -ron 67,2) | Qsp 100 (envi -ron 73,0) | Qsp 100 (envi -ron 57,6) |

Toutes ces compositions ont une texture agréable et souple plus ou moins ferme mais se délitant facilement au contact de l'eau en générant un effet chaleur.

## Revendications

1. Composition cosmétique anhydre comprenant un ou plusieurs agents structurants organiques, plusieurs agents absorbants choisis parmi les mélanges d'un ou plusieurs amidons modifiés et d'une ou plusieurs farines de bois, un ou plusieurs agents tensioactifs et une phase liquide anhydre.

2. Composition selon la revendication 1 **caractérisée en ce que** le ou les agents structurants organiques sont formés d'un ou plusieurs types de particules.

3. Composition selon la revendication 2 **caractérisée en ce que** les particules sont choisies parmi les particules de matériaux thermoplastiques choisis parmi les polyamides, les polymères ou copolymères d'acrylonitrile, de chlorure de vinylidène, de chlorure de vinyle et/ou de monomère acrylique ou styrénique, éventuellement expansés, et les microsphères microporeuses.

4. Composition selon la revendication 3 **caractérisée en ce que** les particules sont choisies parmi les particules creuses d'un copolymère expansé de chlorure de vinylidène et d'acrylonitrile, ou de chlorure de vinylidène, d'acrylonitrile et de méthacrylate de méthyle.

5. Composition selon l'une quelconque des revendications précédentes **caractérisée en ce que** le ou les agents structurants organiques représentent de 1 à 10%, de préférence de 2 à 6%, de préférence encore de 2,5 à 5%, mieux de 3 à 4% en poids du poids total de la composition cosmétique.

6. Composition selon l'une quelconque des revendications précédentes **caractérisée en ce que** le ou les amidons modifiés sont des amidons de pomme de terre.

7. Composition selon l'une quelconque des revendications précédentes **caractérisée en ce que** la farine de bois est la farine d'épicéa.

8. Composition selon l'une quelconque des revendications précédentes **caractérisée en ce que** les agents absorbants représentent de 3 à 50%, de préférence de 5 à 35%, mieux de 10 à 20%, en poids du poids total de la composition.

9. Composition selon l'une quelconque des revendications précédentes **caractérisée en ce que** le ou les amidons modifiés représentent de 3 à 25%, mieux de 4 à 15% en poids du poids total de la composition.

10. Composition selon l'une quelconque des revendications précédentes **caractérisée en ce que** la ou les farines de bois représentent de 1 à 25%, de préférence de 1,5 à 15% en poids du poids total de la composition.

11. Composition selon l'une quelconque des revendications précédentes **caractérisée en ce que** le ou les agents tensioactifs sont choisis parmi les tensioactifs cationiques, anioniques, non ioniques ou amphotères.

12. Composition selon la revendication précédente **caractérisée en ce que** le ou les tensioactifs représentent de 1 à 40 %, de préférence de 2 à 20%, mieux de 4 à 20%, en poids du poids total de la composition.

13. Composition selon l'une quelconque des revendications précédentes **caractérisée en ce que** la phase liquide anhydre comprend un ou plusieurs composés choisis parmi les manoalcools, les polyols et les corps gras.

14. Composition selon l'une quelconque des revendications précédentes **caractérisée en ce que** la phase liquide anhydre représente au moins 50%, de préférence de 50 à 95% en poids du poids total de la composition.

15. Utilisation d'une composition telle que définie dans l'une quelconque des revendications précédentes pour le nettoyage et/ou le conditionnement des matières kératiniques.

## Patentansprüche

1. Wasserfreie kosmetische Zusammensetzung, umfassend ein oder mehrere organische Strukturierungsmittel, mehrere Absorptionsmittel, die aus Mischungen aus einer oder mehreren modifizierten Stärken und einem oder mehreren Holzmehlen ausgewählt sind, ein oder mehrere Tenside und eine wasserfreie flüssige Phase.

2. Zusammensetzung nach Anspruch 1, **dadurch gekennzeichnet, dass** das organische Strukturierungsmittel bzw. die organischen Strukturierungsmittel aus einem oder mehreren Typen von Teilchen gebildet ist bzw. sind.

3. Zusammensetzung nach Anspruch 2, **dadurch gekennzeichnet, dass** die Teilchen aus Teilchen aus thermoplastischen Materialien, die aus Polyamiden, Polymeren oder Copolymeren von Acrylnitril, Vinylidenchlorid, Vinylchlorid und/oder Acryl- oder Styrol-Monomer ausgewählt sind, die gegebenenfalls expandiert sind, und mikroporösen Mikrokugeln ausgewählt sind.

4. Zusammensetzung nach Anspruch 3, **dadurch gekennzeichnet, dass** die Teilchen aus hohlen Teilchen aus einem expandierten Copolymer von Vinylidenchlorid und Acrylnitril oder Vinylidenchlorid, Acrylnitril und Methylmethacrylat ausgewählt sind.

5. Zusammensetzung nach einem der vorhergehenden Ansprüche, **dadurch gekennzeichnet, dass** das organische Strukturierungsmittel bzw. die organischen Strukturierungsmittel 1 bis 10 Gew.-%, vorzugsweise 2 bis 6 Gew.-%, weiter bevorzugt 2,5 bis 5 Gew.-% und noch besser 3 bis 4 Gew.-% des Gesamtgewichts der kosmetischen Zusammensetzung ausmacht bzw. ausmachen.

6. Zusammensetzung nach einem der vorhergehenden Ansprüche, **dadurch gekennzeichnet, dass** es sich bei der modifizierten Stärke bzw. den modifizierten Stärken um Kartoffelstärken handelt.

7. Zusammensetzung nach einem der vorhergehenden Ansprüche, **dadurch gekennzeichnet, dass** es sich bei dem Holzmehl um Fichtenmehl handelt.

8. Zusammensetzung nach einem der vorhergehenden Ansprüche, **dadurch gekennzeichnet, dass** die Absorptionsmittel 3 bis 50 Gew.-%, vorzugsweise 5 bis 35 Gew.-% und noch besser 10 bis 20 Gew.-% des Gesamtgewichts der Zusammensetzung ausmachen.

9. Zusammensetzung nach einem der vorhergehenden Ansprüche, **dadurch gekennzeichnet, dass** die modifizierte Stärke bzw. die modifizierten Stärken 3 bis 25 Gew.-% und noch besser 4 bis 15 Gew.-% des Gesamtgewichts der Zusammensetzung ausmacht bzw. ausmachen.

10. Zusammensetzung nach einem der vorhergehenden Ansprüche, **dadurch gekennzeichnet, dass** das Holzmehl bzw. die Holzmehle 1 bis 25 Gew.-% und vorzugsweise 1,5 bis 15 Gew.-% des Gesamtgewichts der Zusammensetzung ausmacht bzw. ausmachen.

11. Zusammensetzung nach einem der vorhergehenden Ansprüche, **dadurch gekennzeichnet, dass** das Tensid bzw. die Tenside aus kationischen, anionischen, nichtionischen oder amphoteren Tensiden ausgewählt ist bzw. sind.

12. Zusammensetzung nach dem vorhergehenden Anspruch, **dadurch gekennzeichnet, dass** das Tensid bzw. die Tenside 1 bis 40 Gew.-%, vorzugsweise 2 bis 20 Gew.-% und noch besser 4 bis 20 Gew.-% des Gesamtgewichts der Zusammensetzung ausmacht bzw. ausmachen.

13. Zusammensetzung nach einem der vorhergehenden Ansprüche, **dadurch gekennzeichnet, dass** die wasserfreie flüssige Phase eine oder mehrere Verbindungen, die aus Monoalkoholen, Polyalkoholen und Fettsubstanzen ausgewählt sind, umfasst.

14. Zusammensetzung nach einem der vorhergehenden Ansprüche, **dadurch gekennzeichnet, dass** die wasserfreie flüssige Phase mindestens 50 Gew.-% und vorzugsweise 50 bis 95 Gew.-% des Gesamtgewichts der Zusammensetzung ausmacht.

15. Verwendung einer Zusammensetzung gemäß einem der vorhergehenden Ansprüche zur Reinigung und/oder Konditionierung von Keratinmaterialien.

## Claims

1. Anhydrous cosmetic composition comprising one or more organic structuring agents, several absorbers chosen from mixtures of one or more modified starches and one or more wood meals, one or more surfactants and an anhydrous liquid phase.

2. Composition according to Claim 1, **characterized in that** the organic structuring agent(s) are formed from one or more types of particle.

3. Composition according to Claim 2, **characterized in that** the particles are chosen from particles of thermoplastic materials chosen from polyamides, polymers or copolymers of acrylonitrile, of vinylidene chloride, of vinyl chloride and/or of acrylic or styrene monomer, optionally expanded, and microporous microspheres.

4. Composition according to Claim 3, **characterized in that** the particles are chosen from hollow particles of an expanded copolymer of vinylidene chloride and of acrylonitrile, or of vinylidene chloride, acrylonitrile and methyl methacrylate.

5. Composition according to any one of the preceding claims, **characterized in that** the organic structuring agent(s) represent from 1% to 10%, preferably from 2% to 6%, more preferably from 2.5% to 5% and better still from 3% to 4% by weight relative to the total weight of the cosmetic composition.

6. Composition according to any one of the preceding claims, **characterized in that** the modified starch(es) are potato starches.

7. Composition according to any one of the preceding claims, **characterized in that** the wood meal is spruce meal.

8. Composition according to any one of the preceding claims, **characterized in that** the absorbers represent from 3% to 50%, preferably from 5% to 35% and better still from 10% to 20% by weight relative to the total weight of the composition.

9. Composition according to any one of the preceding claims, **characterized in that** the modified starch (es) represent from 3% to 25% and better still from 4% to 15% by weight relative to the total weight of the composition.

10. Composition according to any one of the preceding claims, **characterized in that** the wood meal(s) represent from 1% to 25% and preferably from 1.5% to 15% by weight relative to the total weight of the composition.

11. Composition according to any one of the preceding claims, **characterized in that** the surfactant(s) are chosen from cationic, anionic, nonionic and amphoteric surfactants.

12. Composition according to the preceding claim, **characterized in that** the surfactant(s) represent from 1% to 40%, preferably from 2% to 20% and better still from 4% to 20% by weight relative to the total weight of the composition.

13. Composition according to any one of the preceding claims, **characterized in that** the anhydrous liquid phase comprises one or more compounds chosen from monoalcohols, polyols and fatty substances.

14. Composition according to any one of the preceding claims, **characterized in that** the anhydrous liquid phase represents at least 50% and preferably from 50% to 95% by weight relative to the total weight of the composition.

15. Use of a composition as defined in any one of the preceding claims for cleansing and/or conditioning keratin materials.
